Europäisches Patentamt

European Patent Office

Office européen des brevets

(11) Veröffentlichungsnummer: **0 493 701 A2**

## (12) EUROPÄISCHE PATENTANMELDUNG

(21) Anmeldenummer: **91120927.8**

(51) Int. Cl.5: **A61C 3/02**

(22) Anmeldetag: **06.12.91**

(30) Priorität: **08.12.90 DE 9016647 U**

(43) Veröffentlichungstag der Anmeldung:
**08.07.92 Patentblatt 92/28**

(84) Benannte Vertragsstaaten:
**CH DE FR IT LI**

(71) Anmelder: **Gebr. Brasseler GmbH & Co. KG**
**Trophagener Weg 25**
**W-4920 Lemgo(DE)**

(72) Erfinder: **Stölting, Horst**
**Zum Riessen 3**
**W-4925 Kalletal-Kalle(DE)**

(74) Vertreter: **Hoefer, Theodor, Dipl.-Ing.**
**Kreuzstrasse 32**
**W-4800 Bielefeld 1(DE)**

(54) **Fräser für die Kieferchirurgie.**

(57) Bei einem Fräser mit einer Drallverzahnung insbesondere Sägeverzahnung ist eine Kreuzverzahnung (11) mit einer Grundverzahnung (12) mit einem kleineren Drallwinkel gegenüber seiner Längsachse und einr damit kombinierten weiteren Verzahnung (13) mit einem größeren Drallwinkel gegenüber seiner Längsachse.Dabei kann die Grundverzahnung 10 bis 15 Grad und die weitere Verzahnung unter einem Winkel von 35 bis 45 Grad verlaufen.

Fig. 1

EP 0 493 701 A2

Die Erfindung bezieht sich auf einen Fräser, insbesondere zur kieferchirurgischen Behandlung von Knochen o. dgl.

Derartige spanabhebende Werkzeuge sind mit einer Drallverzahnung ausgestattet, die als ausgeprägte Sägeverzahnung gebildet sein kann.

In derPraxis haben sich derartige Fräser gut bewährt, verursachen jedoch nachteilige Vibrationen und lassen sich nicht immer kontrolliert führen.

Aufgabe der Erfindung ist es, derartige drallverzahnte Fräser gem. vorgenannter Gattung in ihrer Ausbildung und damit auch in ihrer Anwendung zu verbessern und dabei auch die Arbeit mit einem solchen Fräser zu vereinfachen.

Diese Aufgabe wird bei einem Fräser der vorgenannten Gattung durch eine Kreuzverzahnung gelöst, bei der eine Grundverzahnung mit einem kleineren Drallwinkel gegenüber seiner Längsachse und einer damit kombinierten weiteren Verzahnung mit einem größeren Drallwinkel gegenüber seiner Längsachse ausgestattet ist.

Bei einer bevorzugten Ausführungsform kann die Grundverzahnung in einem Winkel von 10 bis 15 Grad verlaufen, die weitere Verzahnung hat vorzugsweise einen größeren Drallwinkel gegenüber seiner Längsrichtung und zwar unter einem Winkel von 35° bis 45°.

Ein derartiger Fräser ist vorzugsweise konisch. Dabei können der Schneidenschaft (Kopf) und Schneiden bevorzugt aus Sinterhartmetall bestehen.

Als Werkstoffe können aber auch rostfreier Stahl, Werkzeugstahl bzw. Schnellarbeitsstahl verwendet werden.

Weitere Merkmale ergeben sich aus den Unteransprüchen.

Der Schutz erstreckt sich nicht nur auf die Einzelmerkmale, sondern auch auf deren Kombination.

Ein Ausführungsbeispiel der Erfindung ist in der Zeichnung dargestellt und wird im folgenden näher beschrieben. Es zeigen:

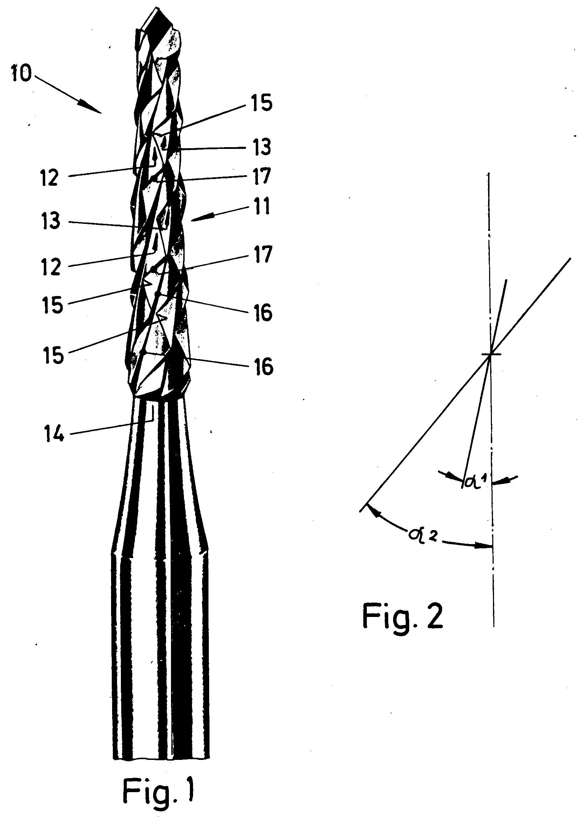

Fig. 1     eine Ansicht eines Knochenfräsers im rechten Winkel zu dessen Längsrichtung;

Fig. 2     eine schematische Darstellung der Abwicklung von zwei sich kreuzenden Verzahnungsreihen desselben Knochenfräsers.

Ein Fräswerkzeug für eine z. B. kieferchirurgische Behandlung von Knochen als sogenannter Knochenfräser (10) ist in der Form eines drallverzahnten Fräsers mit einer Kreuzverzahnung (11) versehen, die eine Grundverzahnung (12) und eine zweite überlagernde Verzahnung (13) aufweist.

Die Grundverzahnung (12) verläuft in einem Winkel von 10 bis 15°, vorzugsweise 12°; diese entspricht einer Steigung von 2,5 mm gegen die Längsachse des Fräsers; die zweite Verzahnung (13) hingegen verläuft in einem Winkel von z. B. 35 bis 45°, vorzugsweise 40°, diese entspricht einer Steigung von 1, 1 mm gegen die Längsachse des Fräsers; diese Kreuzverzahnung (11) wird somit durch einen starken kleineren Drallwinkel der Grundverzahnung (11) in entsprechender Kombination mit einem schwächeren (größeren) Drallwinkel der zweiten Verzahnung (13) erreicht.

Die Verzahnungsreihen der Grundverzahnung (12) sind im Querschnitt in etwa V-förmig in den Schneidenschaft (14) des Knochenfräsers (10) eingearbeitet, wobei der Grund dieser Verzahnungsreihen nicht intermittierend, sondern "durchgehend" ausgebildet ist. Die Verzahnungsreihen der zweiten Verzahnung (13) sind ebenfalls im Querschnitt in etwa V-förmig in den Schneidenschaft (14) des Knochenfräsers (10) eingearbeitet, wobei der Grund dieser Verzahnungsreihen desgleichen nicht intermittierend sondern auch "durchgehend" ausgebildet ist.

Sowohl der Grund der im Querschnitt in etwa V-förmig ausgebildeten Grundverzahnung (12) als auch der zweiten etwa V-förmigen Verzahnung (13) liegen auf einer gedachten Umfangsebene des Schneidenschaftes (14) und weisen im Kreuzungsbereich von Grundverzahnung (12) mit Verzahnung (13) jeweils einen gemeinsamen Tangentialpunkt (15) auf.

Durch die sich mit der Grundverzahnung (12) kreuzenden (überlagernden) Verzahnung (13) sind beider Grundverzahnung (12) einzelne Schneidenabschnitte (16), bei der zweiten Verzahnungsreihe (13) einzelne Schneidenabschnitte (17) gebildet.

Diese Primär- und Sekundärverzahnungen unter verschiedenen Winkeln verlaufen in selben Quadranten.

Durch die beschriebene spezielle Schneidengeometrie des Knochenfräsers (10) ist nicht nur ein ausreichender Spanraum für abzutransportierende Spanmengen geschaffen, sondern auch eine gegenüber den herkömmlichen Knochenfräsern verbesserte Standzeit geboten. Außerdem trägt der Fräser den Knochen ohne größere Kraftanwendung ab und verursacht auch keine nachteiligen Vibrationen o. dgl., wodurch sich der Knochenfräser entsprechend kontrollierter führen läßt.

Der Fräser ist vorzugsweise konisch ausgebildet.

Kopf und Schneiden können bevorzugt aus Sinterhartmetall (Wolframcarbid-Kobalt-Basis) bestehen.

**Patentansprüche**

1.   Fräser mit einer Drallverzahnung insbesondere Sägeverzahnung, gekennzeichnet durch eine Kreuzverzahnung (11) mit einer Grundverzahnung (12) mit einem kleineren Drallwinkel gegenüber seiner Längsachse und einer damit kombinierten weiteren Verzahnung (13) mit einem größeren Drallwinkel gegenüber seiner Längsachse.

2. Fräser nach Anspruch 1, dadurch gekennzeichnet, daß die Grundverzahnung (12) in einem Winkel von 10 bis 15 Grad verläuft.

3. Fräser nach Anspruch 2, dadurch gekennzeichnet, daß die Grundverzahnung (12) in einem Winkel von 12 Grad verläuft.

4. Fräser nach Anspruch 1, dadurch gekennzeichnet, daß die weitere Verzahnung (13) unter einem Winkel von 35 bis 45 Grad verläuft.

5. Fräser nach Anspruch 4, dadurch gekennzeichnet, daß die weitere Verzahnung (13) in einem Winkel von 40 Grad verläuft.

6. Fräser nach einem der Ansprüche 1 bis 5, dadurch gekennzeichnet, daß Kopf (14) und Schneiden aus Sintermetall bestehen.

7. Fräser nach Anspruch 6, dadurch gekennzeichnet, daß das Sinterhartmetall auf Basis Wolfram-Karbid-Kobalt gebildet ist.

8. Fräser nach einem der Ansprüche 1 bis 5, dadurch gekennzeichnet, daß Kopf (14) und Schneiden aus rostfreiem Stahl bestehen.

9. Fräser nach einem der Ansprüche 1 bis 8, dadurch gekennzeichnet, daß der Fräser konisch ausgebildet ist.

10. Fräser nach einem der Ansprüche 1 bis 9, dadurch gekennzeichnet, daß die Verzahnungen V-förmig in den Schneidenschaft (14) eingearbeitet sind.

Fig. 1

Fig. 2

4